# FASCICULE DE BREVET EUROPEEN

(11) **EP 3 243 593 B1**
(45) Date de publication et mention de la délivrance du brevet: **08.04.2020**
(21) Numéro de dépôt: 17170547.8
(22) Date de dépôt: 11.05.2017
(51) Int. Cl.: B23K 1/20, A61N 1/375, B23K 1/00, B23K 1/19, B23K 35/30, B23K 31/12, C22F 1/18, B23K 101/34, B23K 103/14, B23K 103/16, B23K 103/00, B23K 101/30

(54) **PROCÉDÉ DE BRASAGE D'UN ÉLÉMENT METALLIQUE SUR UNE PIÈCE DE ZIRCONE ET DISPOSITIF IMPLANTABLE BRASÉ**
LÖTVERFAHREN EINES METALLELEMENTS AUF EIN ZIRKONOXID-TEIL, UND GELÖTETE IMPLANTIERBARE VORRICHTUNG
METHOD FOR BRAZING A METAL ELEMENT ON A PIECE OF ZIRCONIA AND BRAZED IMPLANTABLE DEVICE

(30) Priorité: 11.05.2016 FR 1654208
(43) Date de publication de la demande: 15.11.2017
(73) Titulaire: PNL HOLDING, 77090 Collegien (FR)
(72) Inventeur: NUVOLI, Didier, 94600 CHOISY LE ROI (FR); PAQUIN, Yvan, 77165 IVERNY (FR)
(74) Mandataire: Lavialle, Bruno François Stéphane

(56) Documents cités:
- EP-A1- 1 542 271
- US-A- 4 225 262

## Description

La présente invention concerne le domaine du brasage d'élément métallique sur une céramique.

### ETAT DE LA TECHNIQUE

Il est connu des dispositifs implantables dans le corps humain, comme les stimulateurs cardiaques ou les implants cochléaires, qui comportent une sonde reliée à un circuit électronique enfermé dans un boîtier en céramique. La céramique utilisée est généralement de l'alumine.

Les normes applicables aux dispositifs implantables dans le corps humain imposent désormais au boîtier de pouvoir résister à des chocs de 2,5 joules.

Pour satisfaire à ces normes, il a été envisagé d'utiliser une céramique plus résistante et plus particulièrement du dioxyde de zirconium ou zircone. Il est apparu que la fabrication de boîtiers en zircone serait cependant problématique. En particulier, la fabrication traditionnelle de boîtiers en alumine comporte des opérations de brasage qui ne sont pas réalisable sur la zircone. Cette solution n'a pas été développée plus avant.

Ce problème du brasage d'un élément métallique sur une pièce en zircone se pose aussi dans d'autres domaines que les celui des dispositifs implantables dans le corps humain.

Il est par ailleurs connu du document US-A-4225262 un procédé de brasage d'un élément en niobium sur une surface d'une pièce de zircone.

### OBJET DE L'INVENTION

Un but de l'invention est de fournir un moyen permettant de braser un élément métallique sur une pièce de zircone.

### BREF EXPOSE DE L'INVENTION

A cet effet, on prévoit, selon l'invention, un procédé de brasage d'un élément métallique sur une surface d'une pièce en zircone, comprenant les étapes de :
- altérer l'état de surface de la pièce pour permettre une accroche d'une première couche de métallisation,
- nettoyer la pièce pour éliminer de sa surface des impuretés,
- déposer sur la surface de la pièce la première couche de métallisation comprenant majoritairement du titane,
- déposer sur la première couche de métallisation une deuxième couche de métallisation comprenant majoritairement du niobium,
- appliquer l'élément contre la deuxième couche de métallisation,
- déposer une brasure d'or sur l'élément et la deuxième couche de métallisation,
- effectuer un refroidissement de la zone brasée de manière pilotée thermiquement,
- un traitement thermique de libération de contrainte étant réalisé sous charge sur l'élément métallique avant le brasage.

L'altération de l'état de surface permet d'en augmenter la rugosité pour favoriser l'accroche de la première couche de métallisation. La première couche de métallisation permet l'accroche de la deuxième couche de métallisation. La deuxième couche de métallisation permet l'accroche de la brasure d'or qui adhère sur l'élément métallique. La libération de contrainte sous charge permet d'éliminer les contraintes accumulées dans l'élément métallique lors de sa fabrication en évitant une déformation de l'élément métallique. De telles contraintes, si elles n'étaient pas éliminées au préalable, pourraient provoquer des fissures dans la zone de brasure lors du refroidissement de celle-ci.

D'autres caractéristiques et avantages de l'invention ressortiront à la lecture de la description qui suit de modes de réalisation particuliers non limitatifs de l'invention.

### BREVE DESCRIPTION DES FIGURES

Il sera fait référence aux dessins annexés, parmi lesquels :
- la figure 1 est une vue partielle en perspective d'un boîtier selon un premier mode de réalisation de l'invention;
- la figure 2 est une vue en coupe d'une variante du premier mode de réalisation de l'invention ;
- les figures 3 et 4 sont des vues de détail des zones III et IV de la figure 2 ;
- la figure 5 est une vue partielle en perspective d'un boîtier selon un deuxième mode de réalisation de l'invention ;
- la figure 6 est une vue de détail de la zone VI de la figure 5.

### DESCRIPTION DETAILLEE DE L'INVENTION

L'invention est ici décrite en application à la fabrication d'un boîtier pour un dispositif implantable dans le corps humain. Bien entendu, l'invention est utilisable pour la fabrication d'autres produits comprenant une partie en zircone sur laquelle doit être fixé un élément en métal.

En référence aux figures, le dispositif de l'invention comprend un boîtier comportant une plaque 10 en zircone, ici une zircone yttriée de type C5. La plaque 10 a ici une forme circulaire.

Au moins un élément métallique est fixé à la plaque de zircone par une brasure.

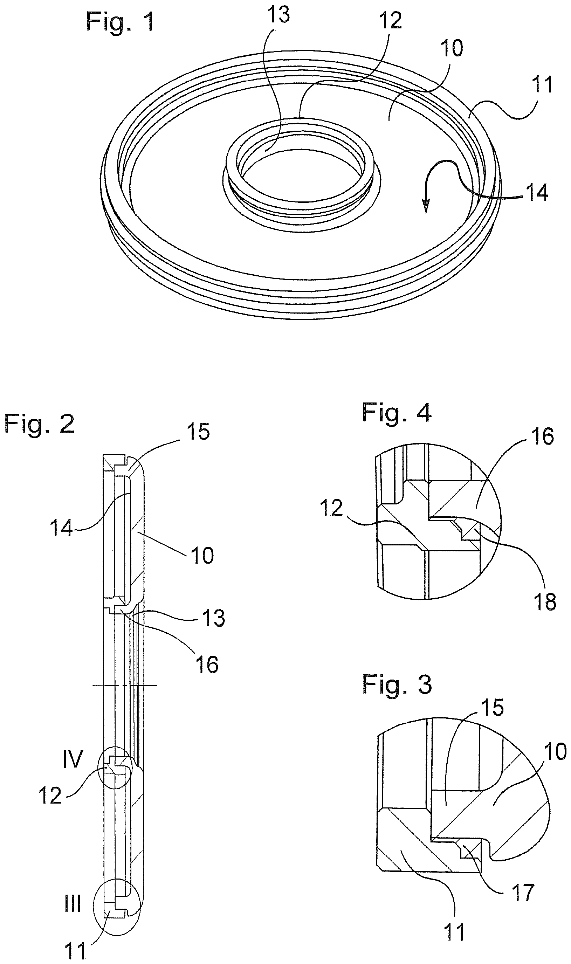

En référence à la figure 1, et selon le premier mode de réalisation, deux éléments métalliques 11, 12 sont fixés à la plaque 10. L'élément métallique 11 est une virole circulaire fixée sur la circonférence externe de la plaque 10. L'élément métallique 12 est une virole circulaire fixée sur le pourtour d'un trou 13 réalisé au centre de la plaque 10. Les éléments métalliques 11, 12 s'étendent tous deux en saillie de la même surface 14 de la plaque 10 et définissent un contour fermé.

Les éléments métalliques 11, 12 sont tous deux en titane.

La brasure qui relie la plaque 10 à chacun des éléments métalliques 11, 12 est continue et permet d'assurer une étanchéité de la liaison entre chacun des éléments métalliques 11, 12 et la plaque 10.

En référence aux figures 2 à 4, et selon la variante du premier mode de réalisation, la plaque 10 comprend un rebord annulaire externe 15 s'étendant en saillie de la surface 14 depuis le bord externe de la plaque 10 et un rebord annulaire interne 16 s'étendant en saillie de la surface 14 autour du trou 13.

L'élément métallique 11 est une virole circulaire dont un rebord annulaire 17 est engagé autour du rebord annulaire externe 15 de la plaque 10. L'élément métallique 12 est une virole circulaire dont un rebord annulaire 18 est engagé à l'intérieur du rebord annulaire interne 16 de la plaque 10.

Comme précédemment, la brasure qui relie la plaque 10 à chacun des éléments métalliques 11, 12 est continue et permet d'assurer une étanchéité de la liaison entre chacun des éléments métalliques 11, 12 et la plaque 10.

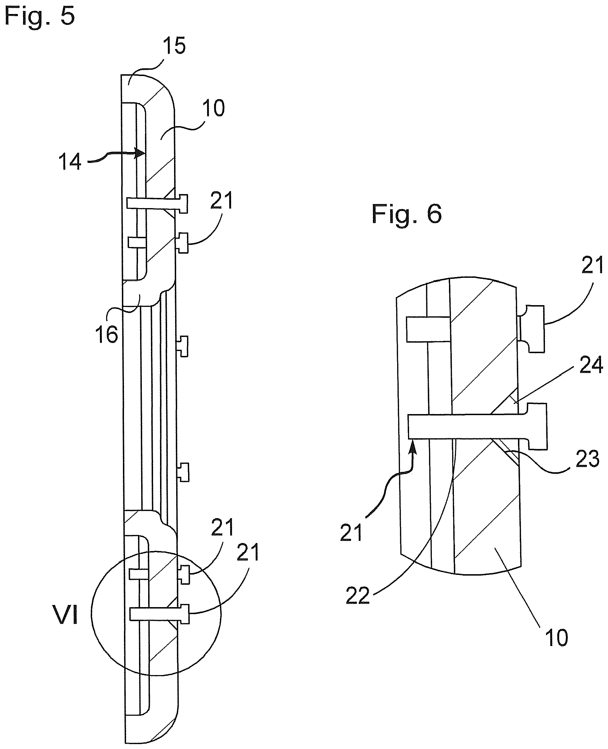

En référence aux figures 5 et 6, et selon le deuxième mode de réalisation, la plaque 10 a une forme sensiblement identique à celle de la plaque 10 de la figure 2.

Des éléments métalliques 21 formant des broches de conduction électrique, traversent la plaque 10 dans la zone annulaire s'étendant entre les rebords 15, 16. Plus précisément, les éléments métalliques 21 ont chacun un tronçon central reçu dans un perçage 22 qui est ménagé dans ladite zone de la plaque 10 et qui débouche par un chanfrein 23 sur une surface de la plaque 10 opposée à la surface 14. Une brasure 24 s'étend dans le chanfrein 23 et entre la paroi du perçage 22 et la surface extérieure de chaque élément conducteur 21. La brasure 24 s'étend de façon continue le long du pourtour de chaque perçage 22 et de l'élément métallique 21 correspondant pour assurer l'étanchéité de la traversée des éléments métalliques 21.

Le matériau des éléments métalliques 21 est ici un alliage de platine et d'iridium.

Le brasage des éléments métalliques 11, 12, 21 sur les plaques 10 sont réalisés au moyen du procédé de brasage selon l'invention.

Ce procédé de brasage va maintenant être décrit.

Selon l'invention, le procédé de brasage débute par des étapes préparatoires visant à favoriser l'accrochage ultérieur de la brasure sur la plaque 10.

Il est ainsi procédé un sablage visant à altérer l'état de surface de la plaque pour en augmenter la rugosité et permettre une accroche d'une première couche de métallisation. Le sablage est réalisé sur la surface destinée à recevoir la première couche de métallisation.

La rugosité souhaitée est d'environ 0,4 µm.

La plaque est ensuite traitée pour éliminer de sa surface les impuretés.

Ce traitement comprend :
- une opération de nettoyage à l'acétone, puis
- une opération de grillage effectuée à une température de 1000°C environ.

Ceci permet de nettoyer la surface de la plaque et d'en éliminer les impuretés organiques.

La première couche de métallisation est ensuite déposée sur la surface de la plaque 10 qui sera en contact avec la brasure. La première couche de métallisation comprend majoritairement du titane. Plus précisément ici, la première couche de métallisation comprend environ 100% de titane. L'épaisseur de la première couche de métallisation est comprise entre 4 et 8 µm environ.

Une deuxième couche de métallisation est ensuite déposée sur la première couche de métallisation. La deuxième couche de métallisation comprend majoritairement du niobium. Plus précisément ici, la deuxième couche de métallisation comprend environ 100% de niobium. L'épaisseur de la deuxième couche de métallisation est comprise entre 4 et 8 µm environ.

En parallèle des étapes préparatoires de la plaque, un traitement thermique de libération de contrainte est réalisé sous charge sur l'élément métallique.

Ceci permet une libération des contraintes résultant de l'usinage, sans provoquer de déformation de l'élément métallique.

Le brasage peut ensuite être réalisé.

On commence par appliquer l'élément métallique contre la deuxième couche de métallisation puis on dépose la brasure d'or fondu sur l'élément et la deuxième couche de métallisation.

Un refroidissement de la zone brasée est ensuite effectué de manière pilotée thermiquement. Le pilotage vise ici à effectuer un refroidissement rapide jusqu'à 900°C puis un refroidissement de 3°C par minute jusqu'à 250°C. Le refroidissement est ensuite naturel jusqu'à la température ambiante.

Une série de tests est ensuite effectuée pour s'assurer de la qualité de la soudure.

Le procédé comprend ainsi :
- une étape de vérification de l'étanchéité de la brasure, et
- une étape de soumettre l'assemblage brasé à des chocs thermiques et une étape de vérification de l'étanchéité de la brasure. Les chocs thermiques sont réalisés entre une température inférieure de - 55 °C environ et une température supérieure de + 150 °C environ.

La mise en œuvre du procédé de l'invention est réalisée en utilisant un four permettant une répartition homogène de la chaleur de manière à avoir une dispersion thermique inférieure ou égale à 2° C sur la surface de la plaque.

Bien entendu, l'invention n'est pas limitée aux modes de réalisation décrits mais englobe toute variante entrant dans le champ de l'invention telle que définie par les revendications.

En particulier, l'invention est utilisable avec d'autres zircones et d'autres métaux que ceux mentionnés.

Le traitement thermique de libération de contrainte pourrait être réalisé après que l'élément a été appliqué contre la deuxième couche de métallisation.

Il est possible de réaliser également des tests de déverminage et de vieillissement.

## Revendications

1. Procédé de brasage d'un élément métallique (11, 12) sur une surface d'une pièce en zircone (10), comprenant les étapes de :
- altérer l'état de surface de la pièce pour permettre une accroche d'une première couche de métallisation,
- nettoyer la pièce pour éliminer de sa surface les impuretés,
- déposer sur la surface de la pièce une première couche de métallisation comprenant majoritairement du titane,
- déposer sur la première couche de métallisation une deuxième couche de métallisation comprenant majoritairement du niobium,
- appliquer l'élément contre la deuxième couche de métallisation,
- déposer une brasure d'or (24) sur l'élément et la deuxième couche de métallisation,
- effectuer un refroidissement de la zone brasée de manière pilotée thermiquement,
- un traitement thermique de libération de contrainte étant réalisé sous charge sur l'élément métallique avant le brasage.

2. Procédé selon la revendication 1, dans lequel l'altération d'état de surface comprend un sablage.

3. Procédé selon la revendication 2, dans lequel la surface après altération a une rugosité d'environ 0,4 µm.

4. Procédé selon l'une quelconque des revendications précédentes, dans lequel le nettoyage comprend une opération de grillage.

5. Procédé selon la revendication 4, dans lequel le grillage est effectué à une température de 1000° environ.

6. Procédé selon l'une quelconque des revendications précédentes, dans lequel la première couche de métallisation comprend environ 100% de titane.

7. Procédé selon l'une quelconque des revendications précédentes, dans lequel la deuxième couche de métallisation comprend environ 100% de niobium.

8. Procédé selon l'une quelconque des revendications précédentes, dans lequel l'élément est en titane.

9. Procédé selon l'une quelconque des revendications précédentes, dans lequel l'élément comprend du platine et de l'iridium.

10. Procédé selon l'une quelconque des revendications précédentes, dans lequel la pièce est une plaque et l'élément a un contour fermé, la brasure étant continue le long de l'élément et de la pièce, le procédé comprenant une étape ultérieure de vérification de l'étanchéité de la brasure.

11. Procédé selon la revendication 10, dans lequel la plaque comprend un rebord annulaire (15, 16) pour être fixé à l'élément par la brasure.

12. Procédé selon la revendication 10, comprenant ultérieurement une étape de soumettre l'assemblage brasé à des chocs thermiques et une étape de vérification de l'étanchéité de la brasure.

13. Procédé selon la revendication 12, dans lequel les chocs thermiques sont réalisés entre une température inférieure de - 55 °C environ et une température supérieure de + 150 °C environ.

14. Procédé selon la revendication 1, dans lequel le traitement thermique de libération de contrainte étant réalisé sous charge sur l'élément métallique est effectué avant que l'élément ne soit appliqué contre la deuxième couche de métallisation.

15. Dispositif implantable dans le corps humain, comprenant un boîtier comportant une plaque (10) de zircone sur laquelle une virole (11, 12) en titane est brasée au moyen du procédé selon l'une quelconque des revendications précédentes.

## Patentansprüche

1. Verfahren zum Löten eines Metallelements (11, 12) auf eine Oberfläche eines Zirkoniumdioxidteils (10), umfassend die Schritte:
- Ändern des Oberflächenzustandes des Teils, um eine Befestigung einer ersten Metallisierungsschicht zu ermöglichen,
- Reinigen des Teils, um von seiner Oberfläche die Verunreinigungen zu entfernen,
- Aufbringen einer ersten Metallisierungsschicht, die mehrheitlich Titan enthält, auf die Oberfläche des Teils,
- Aufbringen einer zweiten Metallisierungsschicht, die mehrheitlich Niob enthält, auf die erste Metallisierungsschicht,
- Anlegen des Elements gegen die zweite Metallisierungsschicht,
- Aufbringen von Goldlot (24) auf das Element und die zweite Metallisierungsschicht,
- Kühlen der gelöteten Zone auf thermisch gesteuerte Weise,
- wobei eine Entspannungswärmebehandlung unter Last an dem Metallelement vor dem Löten durchgeführt wird.

2. Verfahren nach Anspruch 1, bei dem die Änderung des Oberflächenzustandes ein Sandstrahlen umfasst.

3. Verfahren nach Anspruch 2, bei dem die Oberfläche nach der Änderung eine Rauheit von ungefähr 0,4 µm hat.

4. Verfahren nach einem der vorhergehenden Ansprüche, bei dem die Reinigung einen Röstvorgang umfasst.

5. Verfahren nach Anspruch 4, bei dem das Rösten bei einer Temperatur von ungefähr 1000° erfolgt.

6. Verfahren nach einem der vorhergehenden Ansprüche, bei dem die erste Metallisierungsschicht ungefähr 100 % Titan umfasst.

7. Verfahren nach einem der vorhergehenden Ansprüche, bei dem die zweite Metallisierungsschicht ungefähr 100% Niob umfasst.

8. Verfahren nach einem der vorhergehenden Ansprüche, bei dem das Element aus Titan ist.

9. Verfahren nach einem der vorhergehenden Ansprüche, bei dem das Element Platin und Iridium umfasst.

10. Verfahren nach einem der vorhergehenden Ansprüche, bei dem das Teil eine Platte ist und das Element eine geschlossene Kontur hat, wobei die Lötung kontinuierlich entlang des Elements und des Teils ist, wobei das Verfahren einen späteren Schritt der Überprüfung der Dichtheit der Lötung umfasst.

11. Verfahren nach Anspruch 10, bei dem die Platte einen ringförmigen Rand (15, 16) umfasst, um durch das Lot an dem Element befestigt zu werden.

12. Verfahren nach Anspruch 10, später umfassend einen Schritt des Aussetzens der Lötverbindung gegenüber Wärmeschocks und einen Schritt der Überprüfung der Dichtheit der Lötung.

13. Verfahren nach Anspruch 12, bei dem die Wärmeschocks zwischen einer unteren Temperatur von ungefähr - 55 °C und einer oberen Temperatur von ungefähr + 150 °C erfolgen.

14. Verfahren nach Anspruch 1, bei dem die Entspannungswärmebehandlung, die unter Last an dem Metallelement durchgeführt wird, erfolgt, ehe das Element gegen die zweite Metallisierungsschicht angelegt wird.

15. Vorrichtung, die in den menschlichen Körper implantierbar ist, umfassend ein Gehäuse, das eine Zirkoniumdioxidplatte (10) umfasst, auf die ein Titanring (11, 12) mittels des Verfahrens nach einem der vorhergehenden Ansprüche gelötet wird.

## Claims

1. A method for brazing a metal part (11, 12) onto a surface of a zirconia component (10), comprising the steps of:
- altering the surface state of the component to permit the attachment of a first metallization layer,
- cleaning the component to eliminate the impurities from its surface,
- depositing a first metallization layer, consisting mainly of titanium, on the surface of the component,
- depositing a second metallization layer, consisting mainly of niobium, on the first metallization layer,
- applying the part against the second metallization layer,
- depositing a gold brazing metal (24) on the part and the second metallization layer, and
- cooling the brazed area in a temperature-controlled manner,
- stress-relieving heat treatment being performed under load on the metal part before brazing.

2. The method as claimed in claim 1, wherein the alteration of surface state comprises sandblasting.

3. The method as claimed in claim 2, wherein the surface after alteration has a roughness of approximately 0.4 µm.

4. The method as claimed in any of the preceding claims, wherein the cleaning step comprises a burning operation.

5. The method as claimed in claim 4, wherein the burning is carried out at a temperature of approximately 1000°.

6. The method as claimed in any of the preceding claims, wherein the first metallization layer comprises approximately 100% titanium.

7. The method as claimed in any of the preceding claims, wherein the second metallization layer comprises approximately 100% niobium.

8. The method as claimed in any of the preceding claims, wherein the part is made of titanium.

9. The method as claimed in any of the preceding claims, wherein the part comprises platinum and iridium.

10. The method as claimed in any of the preceding claims, wherein the component is a plate and the part has a closed contour, the brazing metal being continuous along the part and the component, the method comprising a further step of checking the fluid-tightness of the brazing metal.

11. The method as claimed in claim 10, wherein the plate comprises an annular rim (15, 16) to be fixed to the part by the brazing metal.

12. The method as claimed in claim 10, further comprising a step of subjecting the brazed assembly to thermal shock and a step of checking the fluid-tightness of the brazing metal.

13. The method as claimed in claim 12, wherein the thermal shocks are executed between a lower temperature of approximately -55°C and an upper temperature of approximately +150°C.

14. The method as claimed in claim 1, wherein the stress-relieving heat treatment, being performed under load on the metal part before brazing, is carried out before the part is applied against the second metallization layer.

15. Device implantable into the human body, comprising a housing including a plate (10) of zirconia onto which a titanium collar (11, 12) is brazed by the method as claimed in any of the preceding claims.
